# DEMANDE DE BREVET EUROPEEN

(11) **EP 1 312 666 A1**
(43) Date de publication de la demande: **21.05.2003**
(21) Numéro de dépôt: 01870248.0
(22) Date de dépôt: 19.11.2001
(51) Int. Cl.: C12M 1/107

(54) **Procédé de destruction de valeurs mobilières**

(71) Demandeur: Sorghal asbl, 4500 Huy (BE)
(72) Inventeur: Chapelle, Jean, 4560 Pailhe (BE); Bigare, Pascal, 5380 Fernelmont (BE); Marche, Christian, 4550 Villers-Le-Temple (BE); Wauthelet, Marc, 5640 Biesme (BE); Romedenne, Raphael, 5502 Thynes (BE); Hubin, Isabelle, 4100 Seraing (BE); Loix, Sophie, 6230 Orbaix (BE)
(74) Mandataire: Van Malderen, Michel

(57) **Abrégé**

La présente invention se rapporte à un dispositif pour la destruction irréversible de valeurs mobilières préalablement découpées et/ou broyées, caractérisé en ce que ledit dispositif comprend au moins une cuve d'hydrolyse et d'acidogenèse (1), au moins une cuve de méthanisation et d'acétogenèse (2), au moins une unité de mélange et de broyage (3) et au moins une unité d'évacuation munie d'un filtre (4), ainsi qu'au moins une pompe (5) assurant la circulation des fluides dans les tuyaux (6) qui relient les différentes unités entre elles et qui permettent une utilisation en continu dudit dispositif.

## Description

### Objet de l'invention

La présente invention se rapporte à un procédé de destruction irréversible de valeurs mobilières par voie biologique anaérobie ainsi qu'à un dispositif rendant possible ladite destruction.

### Etat de 1a technique

Les banques nationales et de façon plus générale les organismes ou les entreprises chargés de la destruction irréversible de valeurs mobilières sont confrontés actuellement à des fortes contraintes telles que l'élimination des risques de reconnaissance et la dispersion des différents composants rendant impossible la détermination de l'origine des valeurs mobilières.

Par valeurs mobilières, il faut entendre sans être limitatif, les billets de banque, chèques, obligations, bons de caisse, certificats immobiliers, actions et produits dérivés ou tout autre document de valeur au sens général.

Ces valeurs mobilières contiennent aujourd'hui de nombreuses caractéristiques pour lutter contre leur falsification. A titre d'exemple, nous citerons la composition particulière du papier contenant notamment du coton. Ce papier est souvent fabriqué en étant soumis à des pressions extrêmement élevées afin d'y incruster certains éléments. Les encres d'impression ont des caractéristiques tout à fait particulières (encres à couleur changeante, encres incolores, etc.) et les nombreux métaux présents sous forme de fil de sécurité, de chiffres, d'hologrammes ou de surfaces métallisées rendent la falsification par photocopie pratiquement impossible. Il y a également toute une série d'éléments chimiques et inhabituels qui méritent d'être récupérés lors du processus de destruction.

Les mesures de sécurité entourant non seulement la fabrication de ces documents, mais également celles entourant leur destruction sont évidemment draconiennes.

C'est le contexte de ces contraintes qui est à l'origine de la présente invention.

Pour satisfaire aux exigences de sécurité, les responsables de l'élimination de ces valeurs mobilières procèdent généralement en plusieurs étapes.

Dans un premier temps, les documents sont découpés en fines lamelles et ensuite compactés afin d'en réduire le volume pour le transport. Cette étape se fait généralement de manière interne à ces organismes, et ceci sous haute surveillance, pour des raisons évidentes.

Dans un second temps, ces lamelles compactées sont acheminées vers leur lieu de destruction ou de stockage définitif.

Différentes méthodes de destruction de valeurs mobilières existent. Parmi les méthodes utilisées actuellement, on peut citer l'incinération et la mise en décharge.

L'incinération présente l'avantage d'être peu coûteuse mais entraîne évidemment l'inconvénient que tous les éléments de valeur sont perdus. Par ailleurs, il y a également le problème des cendres contenant certains composants nécessitant souvent une mise en décharge particulière. Par ailleurs, la composition des encres est souvent très particulière et, pour des raisons évidentes de sécurité, non divulguée. Ceci entraîne une incertitude supplémentaire lors de l'incinération car ces composants peuvent donner lieu à des fumées pouvant atteindre un certain degré de toxicité.

La présente invention se fixe pour objectif de fournir une alternative à l'incinération ou à la mise en décharge de valeurs mobilières.

### Buts de l'invention

La présente invention vise à fournir un procédé et un dispositif de destruction irréversible de valeurs mobilières permettant à la fois d'associer une récupération de composants valorisables et une valorisation énergétique, tout en minimisant les nuisances générées.

### Résumé de l'invention

La présente invention a pour objet de proposer un dispositif pour la destruction irréversible de valeurs mobilières préalablement découpées et/ou broyées caractérisé en ce que ledit dispositif comprend au moins une cuve d'hydrolyse et d'acidogenèse (1), au moins une cuve de méthanisation et d'acétogenèse (2), au moins une unité de mélange et de broyage (3) et au moins une unité d'évacuation munie d'un filtre (4) ainsi qu'au moins d'une pompe (5) assurant la circulation des fluides dans les tuyaux (6) qui relient les différentes unités entre elles et qui permettent une utilisation en continu dudit dispositif.

Un autre aspect de l'invention propose un procédé pour la destruction irréversible de valeurs mobilières préalablement découpées et/ou broyées, caractérisé en ce que lesdites valeurs sont prétraitées dans au moins une cuve d'hydrolyse et d'acidogenèse (1) à une température comprise entre 30 et 40°C et ensuite transférées petit à petit vers une ou plusieurs cuves de méthanisation et d'acétogenèse (2), pour y être digérées en milieu anaérobie à une température comprise entre 50 et 60°C et transformés notamment en méthane et en dioxyde de carbone, la partie transférée étant immédiatement remplacée par du mélange non prétraité provenant de l'unité de broyage et de mélange (3) comprenant lesdites valeurs mobilières.

Une autre caractéristique de l'invention consiste en ce que les effluents de la cuve de méthanisation sont pressés dans un système de filtration (4) afin de séparer la fraction liquide (riche en bactéries et qui sera remise en cuve de méthanisation) et la fraction solide (dans laquelle il sera possible de récupérer des éléments métalliques et d'autres éléments à haute valeur commerciale)

Un autre aspect de la présente invention propose l'utilisation d'une solution inoculante pour l'hydrolyse et l'acidogenèse de valeurs mobilières préalablement découpées et/ou broyées caractérisé en ce que ladite solution est composée de bactéries issues d'une liqueur provenant d'un digesteur méthanique (la liqueur y ayant subi une digestion de 30 jours à 35°C et est constituée de bouses de bovins alimentés par des substrats cellulosiques). Les bactéries peuvent également provenir de liqueur extraite de réacteur anaérobie (au sens large : décanteur, digesteur, ...) pour substrats cellulosiques.

### Brève description des figures

La figure 1 représente le schéma de l'installation pilote traitant les valeurs mobilières préalablement découpées et/ou broyées.

La figure 2 représente l'unité de prétraitement effectuant l'hydrolyse et l'acidogenèse, cette unité est une partie intégrante de l'installation pilote.

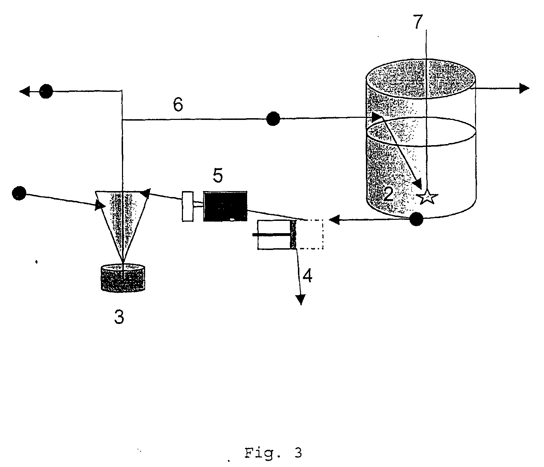

La figure 3 représente l'unité de digestion anaérobie dans laquelle s'effectue l'acétogenèse et la méthanisation, cette unité est également une partie intégrante de l'installation pilote.

### Description détaillée de l'invention

Les valeurs mobilières à détruire sont découpées en fines lamelles et compactées dans un premier stade. Le détail de cette étape de destruction ne sera pas décrit car elle est faite de manière interne à la source.

C'est donc à l'état compacté que les valeurs mobilières doivent être traitées. Dans la mesure où il s'agit de fibres cellulosiques fortement comprimées, on procède alors, de préférence, à un broyage humide de façon à obtenir des particules d'une taille suffisamment petite pour assurer la formation d'une liqueur homogène, mettre en solution le maximum de composants et créer une surface spécifique importante pour l'attaque bactérienne.

Le produit résultant de ce broyage est alors acheminé dans une première cuve de traitement qui est ouverte, appelée cuve d'hydrolyse et d'acidogenèse 1, dans laquelle on procède en milieu aérobie à une première dégradation des éléments contenus dans le produit en le mettant en contact avec une solution inoculée adéquate. Celle-ci va permettre la décomposition (l'hydrolyse) progressive en monomères des polymères biologiques complexes tels que la cellulose, suivi d'une transformation des monomères en acides gras volatils (acétate, propionate, butyrate, pentanate) constituant l'acidogenèse et en substances métabolisables pour les bactéries. Des composants minéraux sont aussi partiellement dégradés.

Lors de cette étape, les monomères formés peuvent être notamment des sucres simples et/ou des acides gras secrétés par des exoenzymes dans le milieu. Cette étape présente l'avantage de liquéfier des substances récalcitrantes par un développement naturel de certaines souches de bactéries (contenues dans l'inoculum ajouté au départ).

L'inconvénient de cette étape est cependant l'acidification de la liqueur de décomposition qui inhibe fortement les bactéries acétogènes et méthanogènes qui devront intervenir lors de la deuxième étape de digestion. L'évolution du pH au cours de ce processus est naturelle et n'est pas influencée par l'opérateur.

Après cette première étape, on passe à l'acétogenèse et à la méthanogenèse en milieu anaérobie dans une cuve fermée 2 où les monomères précédemment cités sont transformés en méthane et en gaz carbonique, notamment.

La séparation des deux étapes en deux cuves distinctes et le contrôle strict des charges (en substrats) permettent d'optimiser la dégradation et d'éviter l'inhibition des bactéries méthanigènes. Le pH dans la cuve 2 devra être maintenu entre 6 et 8 naturellement ou par l'ajout d'urée pour permettre la méthanogenèse.

Le volume utile (volume de liquide à l'intérieur de la cuve) de la cuve 2 est de maximum 1,5 fois supérieur à celui de la cuve 1, ceci afin de maintenir un pH stable dans la cuve 2 et des conditions optimales pour les bactéries. Le temps de séjour des substrats est de 30 jours dans la cuve 1 (il faudra donc ajouter chaque jour un volume de substrat, broyé dans l'eau, correspondant à 3,33% et retirer une quantité équivalente). Dans la cuve 2, le volume correspondant d'alimentation sera donc de 2,22%.
Les substrats broyés ajoutés seront à une concentration de 25g de matière sèche par litre d'eau (potable).

Les conditions de température dans l'étape d'hydrolyse et d'acidogenèse se situent généralement entre 30 et 40°C, de préférence aux alentours de 35°C pour permettre le développement optimal des bactéries.

Après un temps de démarrage d'au moins 30 jours, la durée exacte étant fortement conditionnée par le produit traité, on prélève alors journellement une quantité correspondant à environ un trentième, c'est à dire 3,3% de la première cuve de prétraitement 1 pour la transférer dans la deuxième cuve 2 étanche à l'air et emplie au départ d'un inoculum riche en bactéries méthanigènes (par exemple par l'effluent d'un digesteur méthanique agricole alimenté à 35°C par des boues de bovins). Cette cuve est appelée cuve d'acétogenèse et de méthanogenèse 2. Cette cuve est munie d'un débitmètre pour mesurer le débit de gaz.

Dans cette seconde étape, un mélange de bactéries va décomposer les acides gras et les métabolites en acétates et en hydrogène (acétogenèse), et produire de façon simultanée du dioxyde de carbone et du méthane (méthanogenèse) par l'intermédiaire de souches adéquates développées dans des conditions de digestion de cellulose, soit à partir d'acétates, soit à partir de la réduction du dioxyde de carbone par l'hydrogène. Le méthane peut être récupéré pour la valorisation énergétique.

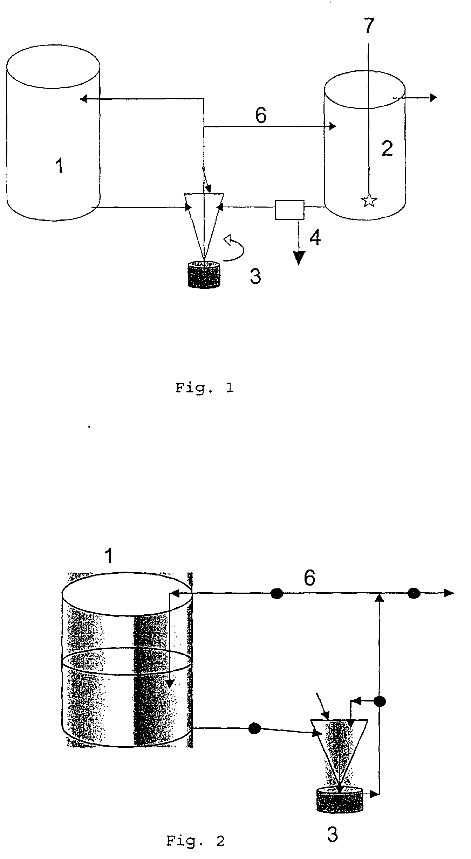

La matière soustraite à la cuve de prétraitement aérobie 1 pour alimenter la cuve 2 est immédiatement remplacée par du produit frais provenant du broyeur humide 3. Cette opération a lieu une fois par jour, ce qui permet au système de tourner en régime semi-continu comme l'illustre le schéma de la figure 1. Les deux cuves fonctionnent selon le mode infiniment mélangé : les quantités ajoutées journellement sont mélangées à l'ensemble de la cuve et les quantités ôtées (effluents) journellement proviennent de la liqueur mélangée dans la cuve.

Les cuves, interconnectées par des tuyaux 6 sont munies de sondes de température, de pH, d'un mélangeur et de serpentins chauffants associés à un système de régulation de température. Le mélangeur 7 est mis en route de façon périodique afin d'assurer une homogénéité parfaite de la liqueur traitée.

Une pompe 5 permet d'assurer la circulation des liqueurs dans le dispositif.

Dès le moment où la cuve d'acétogenèse et de méthanogenèse est remplie d'inoculum, on prélève 2,2% de solution par jour que l'on filtre à travers un filtre adéquat 4. Une partie de la fraction liquide des digestats (effluents) est réutilisée dans la cuve 2.

De façon générale, les bactéries méthanogènes sont très sensibles aux conditions environnementales telles que la température, le pH, la concentration en oxygène, en composants organiques et inorganiques. Elles sont sensibles aux antibiotiques et aux métaux lourds.

### Description d'une forme d'exécution préférée de l'invention

Les valeurs mobilières contiennent de grandes quantités de cellulose. Le coton symbolisé par la formule (C₆H₁₀O₅)ₙ est composé à plus de 90% de cellulose.

L'inoculum utilisé est une liqueur qui a fermenté durant au minimum 30 jours à 35°C provenant d'un digesteur méthanique alimenté par des bouses de bovins eux-mêmes essentiellement alimentés par des substrats cellulosiques (foin, paille, ...).

Les bovins alimentés de la sorte développent des souches bactériologiques capables de digérer efficacement la cellulose.

Plusieurs températures ont également été essayées à la fois pour le prétraitement et le traitement final des liqueurs. Finalement une température approximative de 35°C pour l'étape de prétraitement, c'est à dire l'hydrolyse et l'acidogenèse, et une température approximative de 55°C pour l'étape d'acétogenèse et de méthanogenèse en cuve fermée ont été retenues.

Il s'est également avéré au cours du temps que l'étape de broyage apportait beaucoup en terme d'efficacité de la fermentation et donc de gain de temps. Cette étape a donc toujours été maintenue. Le broyage fin à sec des substrats est avantageux, mais requiert plus d'énergie. Le broyage humide permet de libérer les composants des substrats et de les mettre en solution (et donc de les décomposer ensuite), ce qui peut inhiber le démarrage de la fermentation.

Pour les processus de méthanogenèse et d'acétogenèse, le pH doit être compris entre 6.5 et 8 et le rapport optimal carbone/ azote (C/N) doit être compris entre 16 et 20. Des rapports C/N beaucoup plus élevés peuvent être tolérés par les bactéries, mais en cas de carences fortes, de l'urée sera ajoutée à la liqueur de la cuve 2 (pour obtenir maximum 5% d'azote par rapport au carbone contenu dans la liqueur).

Un jeu de vannes permet de réalimenter en permanence la cuve n°2 par de la liqueur en provenance de la cuve n°1, par l'équivalent de la quantité qui a été soustraite lors de chaque cycle et qui sera d'ailleurs remplacé à la base par la quantité équivalente provenant du broyeur humide.

Au point de départ, dans la cuve de prétraitement n°1 qui a une capacité utile de 200 U (Unités de volume égale à un litre, un décalitre ou un m³), on introduit un mélange constitué de 20 U d'inoculum provenant de digesteurs (de la ferme expérimentale du Centre Technique Agricole de Strée, par exemple, ou d'un autre réacteur anaérobie) et de 180 U d'eau ; le mélange est chauffé à environ 35°C durant plus d'une semaine et on ajoute ensuite 25g de substrat broyé (valeurs mobilières) par U.

Le mélange eau-inoculum-substrat est toujours maintenu à 35°C dans la cuve 1. Après 30 jours (démarrage), 3,3% du volume sont extraits journellement pour être ajoutés à la cuve 2 et 3,3% de mélange substrat broyé (25g/l)frais - eau sont ajoutés journellement. Dans la cuve n°2, au point de départ, il y a 300 litres d'inoculum.

## Revendications

1. Dispositif pour la destruction irréversible de valeurs mobilières préalablement découpées et/ou broyées, **caractérisé en ce que** ledit dispositif comprend au moins une cuve d'hydrolyse et d'acidogenèse (1), au moins une cuve de méthanisation et d'acétogenèse (2), au moins une unité de mélange et de broyage (3) et au moins une unité d'évacuation munie d'un filtre (4) ainsi qu'au moins d'une pompe (5) assurant la circulation des fluides dans les tuyaux (6) qui relient les différentes unités entre elles et qui permettent une utilisation en continu dudit dispositif.

2. Procédé pour la destruction irréversible de valeurs mobilières préalablement découpées et/ou broyées, **caractérisé en ce que** lesdites valeurs sont prétraitées dans au moins une cuve d'hydrolyse et d'acidogenèse (1) à une température comprise entre 30 et 40°C, et ensuite transférées petit à petit vers une ou plusieurs cuves de méthanisation et d'acétogenèse (2), pour y être digérées en milieu anaérobie à une température comprise entre 50 et 60°C, et transformés notamment en méthane et en dioxyde de carbone, la partie transférée étant immédiatement remplacée par du mélange non prétraité provenant de l'unité de broyage et de mélange (3) comprenant lesdites valeurs mobilières.

3. Procédé selon la revendication 2, **caractérisé en ce que** les résidus solides obtenus dans la cuve de méthanisation et d'acétogenèse sont récupérés par un système de filtration (4) et post-traités afin de récupérer les éléments valorisables.

4. Utilisation d'une solution inoculante pour l'hydrolyse et l'acidogenèse, ainsi que pour l'acétogenèse et la méthanogenèse de valeurs mobilières préalablement découpées et/ou broyées, **caractérisé en ce que** ladite solution est composée de bactéries issues d'une liqueur provenant d'une digestion méthanique de minimum 30 jours à 35°C de bouses de bovins ayant été nourris par des aliments riches en cellulose ou issues de réacteurs anaérobies traitant des déchets cellulosiques.

## Revendications modifiées

### Revendications modifiées conformément à la règle 86(2) CBE.

1. Procédé pour la destruction irréversible de valeurs mobilières préalablement découpées et/ou broyées, **caractérisé en ce que** lesdites valeurs sont prétraitées dans au moins une cuve d'hydrolyse et d'acidogenèse (1) maintenue à un pH inférieur à 6 et à une température comprise entre 30 et 60 °C, de préférence entre 30 et 40°C, et ensuite transférés petit à petit vers une ou plusieurs cuves de méthanisation et d'acétogenèse (2) maintenues à une valeur de pH comprise entre 7,2 et 8 à l'aide de substances basiques ou de tampons, pour y être digérées en milieu anaérobie à une température comprise entre 50 et 60°C et transformés notamment en méthane, et en dioxyde de carbone, la partie transférée étant immédiatement remplacée par du mélange non prétraité provenant de l'unité de broyage et de mélange (3) comprenant lesdites valeurs mobilières.

2. Procédé selon la revendication 1, **caractérisé en ce que** les résidus liquides obtenus dans la cuve de méthanisation et d'acétogenèse sont récupérés par un système de décantation et/ou de filtration (4) et post-traités afin de récupérer les éléments valorisables et **en ce que** les résidus solides obtenus dans la cuve de méthanisation et d'acétogenèse (2) sont récupérés par l'unité de décantation et/ou filtration (4) et recirculés au moins partiellement dans la cuve de méthanisation (2).

3. Dispositif en deux étapes pour la destruction irréversible de valeurs mobilières préalablement découpées et/ou broyées **caractérisé en ce que** ledit dispositif comprend au moins une cuve d'hydrolyse et d'acidogenèse (1), au moins une cuve de méthanisation et d'acétogenèse (2), au moins une unité de mélange et de broyage (3) et au moins une unité de séparation et de décantation solide/liquide (4) ainsi qu'au moins une pompe (5) assurant la circulation des fluides dans les tuyaux (6) qui relient les différentes unités entre elles et qui permettent une utilisation en continu dudit dispositif.

4. Utilisation d'une solution inoculante pour l'hydrolyse et l'acidogenèse, ainsi que pour l'acétogenèse et la méthanogenèse de valeurs mobilières préalablement découpées et/ou broyées **caractérisé en ce que** ladite solution est composée de bactéries issues d'une liqueur provenant d'une digestion méthanique de minimum 30 jours à 35°C de bouses de bovins ayant été nourris par des aliments riches en cellulose ou issues de réacteurs anaérobies traitant des déchets cellulosiques.
